# EUROPEAN PATENT SPECIFICATION

(11) **EP 1 278 510 B1**
(45) Date of publication and mention of the grant of the patent: **16.03.2005**
(21) Application number: 01940304.7
(22) Date of filing: 09.04.2001
(51) Int. Cl.: A61K 7/48, C11D 9/26, C11D 9/30, C11D 9/38

(54) **DETERGENT BAR COMPOSITION WITH SKIN LIGHTENING AGENTS AND ORGANIC SUNSCREEN**
STÜCKFÖRMIGE REINIGUNGSMITTELZUSAMMENSETZUNG ENTHALTEND HAUTAUFHELLER UND ORGANISCHE SONNENSCHUTZMITTEL
COMPOSITION DETERGENTE EN PAIN CONTENANT DES AGENTS ECLAIRCISSANTS ET DES FILTRES SOLAIRES ORGANIQUES

(30) Priority: 03.05.2000 IN MU04022000
(43) Date of publication of application: 29.01.2003
(73) Proprietor: UNILEVER PLC, London EC4P 4BQ (GB); UNILEVER N.V., 3013 AL Rotterdam (NL)
(72) Inventor: CHOKAPPA, Dhanraj, Kalyansundaram, Andheri (East), Mumbai 400 099 (IN); DHANUKA, Vinodkumar, Ramniranjan, Andheri (East), Mumbai 400 099 (IN); GOVINDRAM, Coimbatore, Balakrishnan, Andheri (East), Mumbai 400 099 (IN); MHASKAR, Sudhakar, Yeshwant, Andheri (East), Mumbai 400 099 (IN); MHATRE, Subhash, Shivshankar, Andheri (East), Mumbai 400 099 (IN); VATSA, Charu, Rajesh, Andheri (East), Mumbai 400 099 (IN)
(74) Representative: Elliott, Peter William
(86) International application number: PCT/EP2001/004009
(87) International publication number: WO 2001/082884

(56) References cited:
- WO-A-97/27835
- WO-A-99/37280
- GB-A- 2 068 997
- US-A- 4 792 443
- US-A- 5 366 665
- US-A- 5 874 392
- PATENT ABSTRACTS OF JAPAN vol. 014, no. 143 (C-0703), 19 March 1990 (1990-03-19) & JP 02 014296 A (KANSAI PAINT CO LTD), 18 January 1990 (1990-01-18)
- DATABASE WPI Section Ch, Week 199017 Derwent Publications Ltd., London, GB; Class D21, AN 1990-129044 XP002175880 & JP 02 078611 A (KANSAI PAINT CO LTD), 19 March 1990 (1990-03-19)
- DATABASE WPI Section Ch, Week 198950 Derwent Publications Ltd., London, GB; Class A96, AN 1989-367603 XP002175881 & JP 01 275524 A (SANSHO SEIYAKU KK), 6 November 1989 (1989-11-06)
- DATABASE WPI Section Ch, Week 199108 Derwent Publications Ltd., London, GB; Class D24, AN 1991-055094 XP002175882 & JP 03 006299 A (NIPPON OILS & FATS CO LTD), 11 January 1991 (1991-01-11)
- DATABASE WPI Section Ch, Week 198703 Derwent Publications Ltd., London, GB; Class D21, AN 1987-017888 XP002175883 & JP 61 275399 A (MIYAZAKI S), 5 December 1986 (1986-12-05)
- DATABASE WPI Section Ch, Week 200049 Derwent Publications Ltd., London, GB; Class B04, AN 2000-535066 XP002175884 & JP 2000 063888 A (SAKAI N), 29 February 2000 (2000-02-29)

## Description

The present invention relates to a novel system for enhanced delivery of functional ingredients for use in soap/detergent formulations. In particular, the invention is directed to enhancing the deposition of benefit agents such as skin lightening agents onto skin, hair or other substrates from soap/detergent based washing compositions.

Melanin is a polymer synthesised by melanocytes, a cell type present at the dermis-epidermis junction, from an amino acid tyrosine. Tyrosine is acted upon by the enzyme tyrosinase which is the key enzyme in melanogenesis. There have been several reports wherein inhibitors of tyrosinase such as hydroquinone and its derivatives, catechols, mercaptoamines, alpha hydroxy acids and others have been used in cosmetic compostions to regulate skin pigmentation. However only some of these are reversible and non-toxic.

In the melanosomes, contained within melanocytes, melanin is synthesised from the individual monomers in the melanocyte. The melanised melanosome gets transferred to the neighbouring cells called keratinocytes. The keratinocytes divide and differentiate, and thus transport the melanosome to the surface of the skin. The intensity of the skin colour is directly related to the number, the size, the melanin content, the rate of formation and migration/transfer of melanosomes to the keratinocytes.

The conventional skin lightening compositions are based on sunscreens or niacin/niacinamide or other skin lightening agents which are believed to control dispersion of melanosomes or inhibit tyrosinase. Sunscreens alone cannot lighten the skin beyond the natural skin colour, their action on the skin is only to reduce the ingress of ambient ultra violet light into the skin, and they are therefore effective only during the day.

Cosmetic compositions to deliver different benefit agents are prepared using different emulsifying systems and vehicles. These compositions are generally formulated as creams, lotions and other forms that are leave on products.

A vanishing cream base which generally comprises fatty acids and alkali metal soaps is one of the preferred forms of such a cosmetically acceptable vehicle as this gives a highly appreciated matty feel to the skin. However, lactic acid, which is known as a skin lightening agent, cannot be used in vanishing creams since it substantially lowers the pH, and thereby destabilises the base. WO9937280 describes the use of alkali metal and alkaline earth metal salts of α-hydroxy and α-acyloxy-carboxylic acids do also have a skin lightening effect and can be effectively delivered to the skin in a vanishing cream base. This is also a leave on product, and the level of soap in the system is up to 10%.

WO 9818447 and US 5,951,991 (P&G) discuss the problem of delivering conditioning benefit agents, and solve the problem by formulating the product as a substantially dry, disposable, personal cleansing product wherein a lathering surfactant is impregnated onto the substrate, and disclose that other active ingredients such as anti-acne actives, anti-wrinkle, anti-microbials, suncreens etc. can be incorporated in the system. The level of the lathering surfactant is only up to 12.5%.

Skin lightening agents such as niacinamide, lactic acid and others are generally unstable in alkaline pH. Personal wash formulations are soap based and have an alkaline pH, the conditions that prove detrimental to the formulations containing skin lightening agents. Hence it is a problem to formulate wash off products comprising these skin lightening agents. The prior art describes a combination of a water insoluble substrate and a lathering surfactant that is only upto a level of 12.5%.

It has not been possible to deliver skin lightening agents such as niacinamide in personal wash bars processed in a conventional way at substantive levels where the total fatty matter is in the range of 30-80%, as these compounds are unstable in alkaline pH at high temperatures. Generally to deliver benefit agents to skin through wash off products it has been found necessary to utilize a delivery system which often contains a cationic polymer.

It is an advantage of the present invention to be able to provide synergistic soap/detergent formulations which would provide for improved delivery of skin lightening agents onto substrates on which they are applied thereby obtaining maximum effective value of the benefit agents used in such formulations.

Thus, according to one aspect of the present invention there is provided a system of enhanced delivery of skin lightening agents for use in detergent/soap formulations wherein the system comprises 15 to 85% soap, 0.1 to 20% skin lightening agent and 1-5% of an organic sunscreen.

Thus, according to another aspect of the present invention there is provided a system of enhanced delivery of skin lightening agents for use in detergent/soap formulations wherein the system comprises 15 to 85% soap 0.1 to 20% skin lightening agent and 1-5% of an organic sunscreen, wherein the skin lightening agents is incorporated into the soap at temperatures in the range of 20-60°C.

Thus, according to a preferred aspect of the present invention there is provided a system of enhanced delivery of skin lightening agents for use in detergent/soap formulations wherein the system comprises 30 to 85% soap, 0.1 to 20% of a skin lightening agent selected from niacinamide, its precursors or derivatives and and 1-5% of an organic sunscreen, wherein the skin lightening agent is incorporated into the solid soap at temperatures in the range of 20-60°C.

The lightening agents may be selected from niacin, niacinamide, its precursors or derivatives, extracts of placenta, hydroquinone and its derivatives (eg. Arbutin), kojic acid, dicarboxylic acids (azelaic acid, sebacic acid), represented by the formula HOOC-(CₓH_{y})-COOH where x=4 to 20 and y=6 to 40, ascorbic acid and its derivatives, hydroxy acids or their esters (lactic acid, glycolic acid, malic acid, tartaric acid, citric acid etc), ferulic acid retinol and derivatives or any other known skin lightening agents. The skin lightening agents may also be introduced as macro domains during plodding.

The term total fatty matter, usually abbreviated to TFM is used to denote the percentage by weight of fatty acid and triglyceride residues present without taking into account the accompanying cations.

For a soap having 18 carbon atoms, an accompanying sodium cation will generally amount to about 8% by weight. Other cations may be employed as desired, such as for example zinc, potassium, magnesium, alkyl ammonium and aluminium.

The term soap denotes salts of carboxylic fatty acids. The soap may be derived from any of the triglycerides conventionally used in soap manufacture - consequently the carboxylate anions in the soap may contain from 8 to 22 carbon atoms.

The soap may be obtained by saponifying a fat and/or a fatty acid. The fats or oils generally used in soap manufacture may be such as tallow, tallow stearines, palm oil, palm stearines, soya bean oil, fish oil, caster oil, rice bran oil, sunflower oil, coconut oil, babassu oil, palm kernel oil, and others. In the above process the fatty acids are derived from oils/fats selected from coconut, rice bran, groundnut, tallow, palm, palm kernel, cotton seed, soya bean, castor etc. The fatty acid soaps can also be synthetically prepared (e.g. by the oxidation of petroleum or by the hydrogenation of carbon monoxide by the Fischer-Tropsch process). Resin acids, such as those present in tall oil, may be used. Naphthenic acids are also suitable.

Tallow fatty acids can be derived from various animal sources and generally comprise about 1-8% myristic acid, about 21-32% palmitic acid, about 14-31% stearic acid, about 0-4% palmitoleic acid, about 36-50% oleic acid and about 0-5% linoleic acid. A typical distribution is 2.5% myristic acid, 29% palmitic acid, 23% stearic acid, 2% palmitoleic acid, 41.5% oleic acid, and 3% linoleic acid. Other similar mixtures, such as those from palm oil and those derived from various animal tallow and lard are also included.

Coconut oil refers to fatty acid mixtures having an approximate carbon chain length distribution of 8% C₈, 7% C₁₀, 48% C₁₂, 17% C₁₄, 8% C₁₆, 2% C₁₈, 7% oleic and 2% linoleic acids (the first six fatty acids listed being saturated). Other sources having similar carbon chain length distributions, such as palm kernel oil and babassu kernel oil, are included within the term coconut oil.

Organic sunscreen, such as Parsol MCX (a registered trade name of Givaudan Roure and chemically 2-ethyl-hexyl-methoxy cinnamate), Parsol 1789 (chemically known as butyl methoxy benzoylmethane) are incorporated. Other benefit agents may be selected from moisturisers, emollients, humectants, non-soap surfactants, antimicrobial agents and antiageing compounds may also be incorporated. The non-soap surfactants may be anionic, nonionic, cationic, amphoteric/ zwitterionic or a mixture thereof. Examples of moisturisers and humectants include polyols, glycerol, cetyl alcohol, carbopol 934, ethoxylated castor oil, paraffin oils, lanolin and its derivatives. Silicone compounds such as silicone surfactants like DC3225C (Dow Corning) and/or silicone emollients, silicone oil (DC-200 Ex-Dow Corning) may also be included. It is preferred to incorporate 1-5% benefit agents at any step prior to step of milling. Alternatively certain of these benefit agents are introduced as macro domains during plodding.

Other additives such as one or more water insoluble particulate materials such as talc, kaolin, polysaccharides such as starch or modified starches and celluloses may be incorporated.

Minor additives such as perfume, colour, preservatives, opacifiers and other conventional additives at 1 to 2 % by weight can be incorporated.

Personal wash products may be formulated in several forms. One of the examples of a personal wash product is a soap bar, the process of manufacture of which is described herein.

The soap may be prepared by neutralising a suitable blend of fatty acids or saponifying a suitable blend of oils or saponifying a blend of fatty acids and oil. The soap is converted into noodles having water content in the range of 12-18% and to the noodles thus produced minors such as perfume, colour, etc. are admixed. Skin lightening agents are added at this stage to the above mass in the sigma mixer and mixed. The temperature during mixing stage is in the range of 20-60°C. The mixed soap mass is further milled, plodded and stamped to produce bars.

In case of soap made from oil saponification, similar process is followed after recovery of glycerine from the soap mass. The neat soap is spray-dried to reduce moisture and resulting noodles are processed as described above.

Suitable equipment used for the soap making are any one that are used in soap/detergent manufacture and is preferably a high shear kneading mixer. The preferred mixers include ploughshare mixer, mixers with kneading members of Sigma type, multi wiping overlap, single curve or double arm. The double arm kneading mixers can be of overlapping or tangential in design. Alternatively the invention can be carried out in a helical screw agitator vessel or multi head dosing pump/high shear mixer and spray drier combinations as in conventional processing.

Illustrations of a few non-limiting examples are provided by way of example only herein showing comparative results of the composition prepared by the present invention and beyond the invention.

### Examples:

### Process for preparing the formulation:

Soap was prepared by the conventional route to obtain 78% total fatty matter (TFM), to which the minor additives such as electrolytes were added. The soap was converted into noodles having water content in the range of 12-18% and to the noodles thus produced minors such as perfume, colour, etc. were admixed. Skin lightening agents at levels described in Table 1, were added at this stage to the above mass in the sigma mixer and mixed. The temperature during mixing stage was about 40°C. The mixed soap mass was further milled, plodded and stamped to produce bars. In Example 1 no skin lightening agent was added and in Example 4 the skin lightening agent was added along with the electrolytes in the neat soap.

The fairness benefit of the formulations described in Table 1 was assessed by change in the colour of skin over extended product usage, to assess the efficacy of skin lightening agents added to the personal wash product. Two groups with a homogeneous average skin colour were selected, and the difference between the group was not statistically significant. Skin colour of the forearms as well as face of a group of volunteers was assessed on a scale of 1 to 10 before commencing the study. This formed the initial reading. The group of volunteers used the soap made of the experimental or control formulation for bathing over 2 months. The skin colour was then assessed after 2 months. The assessment was carried out visually by a trained expert panel.

The fairness benefit was calculated as the difference between skin colour reading after 2 months and initial reading. The positive difference shows the skin has become lighter in colour

**Table 1**

| Composition %wt. | Ex 1 | Ex 2 | Ex 3 | Ex 4 |
|---|---|---|---|---|
| Soap | 78 | 78 | 78 | 78 |
| Niacinamide | - | 1 | 5 | 5 |
| Minors | 2.5 | 2.5 | 2.5 | 2.5 |
| Talc | 6.5 | 5.5 | 1.5 | 1.5 |
| Water | to 100 | to 100 | to 100 | To 100 |
| Niacinamide (µg) delivered/cm² of skin | - | 2 | 10 | - |
| Fairness benefit* | < 0.1 | 0.2 | 0.6 | < 0.1 |
| • Fairness scale: 0 - fair to 10 - dark. | | | | |

The data presented shows that significant fairness benefit is obtained by using the experimental bars.

## Claims

1. A personal wash detergent composition comprising 15-85% soap 0.1-20% of a skin lightening agent and 1-5% of an organic sunscreen.

2. A personal wash detergent composition according to claim 1 in the form of a personal washing detergent bar.

3. A personal wash detergent composition according to claim 1 or claim 2, wherein the skin lightening agent is incorporated into the composition at a temperature of 20-60°C.

4. A personal wash detergent composition according any of the preceding claims, wherein the skin lightening agent is niacinamide or its precursors or derivatives, extracts of placenta, hydroquinone or derivatives thereof, kojic acid, azelaic acid, sebacic acid, ascorbic acid or derivatives thereof, lactic acid, glycolic acid, malic acid, tartaric acid, citric acid, ferulic acid or retinol or mixtures thereof.

5. A personal wash detergent composition according to claim 4 wherein the skin lightening agent is niacinamide, niacin, or its precursors or derivatives, and is most preferably niacinamide.

6. A personal wash detergent composition according to any one of the preceding claims, wherein the organic sunscreen is 2-ethyl-hexyl-methoxy cinnamate (Parsol MCX(TM)) or butyl methoxy benzoylmethane (Parsol 1789(TM)).

7. A personal wash detergent composition according to any one of the preceding claims, additionally comprising 1-5% by weight of a benefit agent.

8. A cosmetic method of lightening skin comprising applying to the skin and washing with a personal washing composition preferably in the form of a detergent bar according to any of the preceding claims.

9. A method according to claim 8, including the step of subsequently rinsing the skin with water.

## Patentansprüche

1. Körperwaschmittelzusammensetzung, umfassend 15-85% Seife, 0,1-20% eines hautaufhellenden Mittels und 1-5% eines organischen Sonnenschutzmittels.

2. Körperwaschmittelzusammensetzung nach Anspruch 1 in Form eines Körperwaschmittelriegels.

3. Körperwaschmittelzusammensetzung nach Anspruch 1 oder Anspruch 2, worin das hautaufhellende Mittel bei einer Temperatur von 20-60°C in die Zusammensetzung eingearbeitet ist.

4. Körperwaschmittelzusammensetzung nach einem der vorangehenden Ansprüche, worin das hautaufhellende Mittel Niacinamid oder seine Vorstufen oder Derivate, Extrakte von Placenta, Hydrochinon oder Derivate davon, Kojisäure, Azelainsäure, Sebacinsäure, Ascorbinsäure oder Derivate davon, Milchsäure, Glycolsäure, Äpfelsäure, Weinsäure, Zitronensäure, Ferulasäure oder Retinol oder Gemische davon ist.

5. Körperwaschmittelzusammensetzung nach Anspruch 4, worin das hautaufhellende Mittel Niacinamid, Niacin oder seine Vorstufen oder Derivate ist und besonders bevorzugt Niacinamid ist.

6. Körperwaschmittelzusammensetzung nach einem der vorangehenden Ansprüche, worin das organische Sonnenschutzmittel 2-Ethyl-hexyl-methoxycinnamat (Parsol MCX™) oder Butylmethoxybenzoylmethan (Parsol 1789™) ist.

7. Körperwaschmittelzusammensetzung nach einem der vorangehenden Ansprüche, zusätzlich umfassend 1-5 Gewichtsprozent eines Vorteilsmittels.

8. Kosmetisches Verfahren zum Aufhellen von Haut, umfassend Auftragen auf die Haut und Waschen mit einer Körperwaschzusammensetzung, vorzugsweise in Form eines Waschmittelriegels, nach einem der vorangehenden Ansprüche.

9. Verfahren nach Anspruch 8, das den Schritt des anschließenden Spülens der Haut mit Wasser einschließt.

## Revendications

1. Composition détergente pour l'hygiène personnelle comprenant 15-85 % de savon, 0,1-20 % d'un agent éclaircissant cutané et 1-5 % d'un filtre solaire organique.

2. Composition détergente pour l'hygiène personnelle selon la revendication 1 sous la forme d'un détergent en pain pour l'hygiène personnelle.

3. Composition détergente pour l'hygiène personnelle selon la revendication 1 ou la revendication 2, dans laquelle l'agent éclaircissant cutané est incorporé dans la composition à une température de 20-60°C.

4. Composition détergente pour l'hygiène personnelle selon l'une quelconque des revendications précédentes, dans laquelle l'agent éclaircissant cutané est le niacinamide ou ses précurseurs ou dérivés, des extraits de placenta, l'hydroquinone ou ses dérivés, l'acide kojique, l'acide azélaïque, l'acide sébacique, l'acide ascorbique ou ses dérivés, l'acide lactique, l'acide glycolique, l'acide malique, l'acide tartrique, l'acide citrique, l'acide férulique ou le rétinol ou des mélanges de ceux-ci.

5. Composition détergente pour l'hygiène personnelle selon la revendication 4 dans laquelle l'agent éclaircissant cutané est le niacinamide, la niacine, ou ses précurseurs ou dérivés, et est le plus préférentiellement le niacinamide.

6. Composition détergente pour l'hygiène personnelle selon l'une quelconque des revendications précédentes, dans laquelle le filtre solaire organique est le 2-éthyl-hexyl-méthoxycinnamate (Parsol MCX™) ou le butyl-méthoxy-benzoylméthane (Parsol 1789™).

7. Composition détergente pour l'hygiène personnelle selon l'une quelconque des revendications précédentes, comprenant en outre 1-5 % en poids d'un agent bénéfique.

8. Méthode cosmétique d'éclaircissement de la peau comprenant l'application sur la peau et le lavage avec une composition pour l'hygiène personnelle de préférence sous la forme d'un détergent en pain selon l'une quelconque des revendications précédentes.

9. Méthode selon la revendication 8, incluant l'étape de rinçage ultérieur de la peau avec de l'eau.
